# EUROPEAN PATENT APPLICATION

(11) **EP 1 764 415 A1**
(43) Date of publication of application: **21.03.2007**
(21) Application number: 05077143.5
(22) Date of filing: 20.09.2005
(51) Int. Cl.: C12P 7/22

(54) **Microbial production of p-hydroxybenzyl alcohol**

(71) Applicant: Nederlandse Organisatie voor Toegepast-Natuurwetenschappelijk Onderzoek TNO, 2628 VK Delft (NL)
(72) Inventor: van der Werf, Maria Johanna, 6701 BC Wageningen (NL)
(74) Representative: Winckels, Johannes Hubertus F.

(57) **Abstract**

The invention relates to the production of aromatic compounds, in particular to the synthesis of aromatic compounds that are hydroxylated at the p (para)- or 4-position, such as p-hydroxybenzyl alcohol (p-HBA). Provided is a method for providing p-HBA, comprising the steps of providing a host cell which has an increased flux in the biosynthesis of at least one aromatic amino acid, allowing said host cell to produce p-HBA and isolating said p-HBA. The (recombinant) host cell is for example a bacterial cell which shows an increased production level of L-phenylalanine and/or L-tyrosine.

## Description

The invention relates to the production of aromatic compounds, in particular to the synthesis of aromatic compounds that are hydroxylated at the p (para)- or 4-position, such as p-hydroxybenzyl alcohol.

Para-hydroxybenzyl alcohol (p-HBA), also known as 4-hydroxybenzyl alcohol, is of major industrial interest. Not only does it serve as monomer in the synthesis of a wide variety of polymers but it is also an attractive precursor for the manufacture of other commercially important 4-hydroxylated aromatic compounds. p-Hydroxybenzyl alcohol and its derivatives are important as a (bulk) starting material for synthesizing various useful organic compounds, such as pharmaceuticals, agricultural chemicals, and antioxidants.

At present, p-HBA is synthesized chemically via various synthetic routes. Known processes for obtaining p-HBA on an industrial scale include a process comprising reacting phenol and formaldehyde to obtain a mixture of para-hydroxybenzyl alcohol and ortho-hydroxybenzyl alcohol and separating the mixture into each isomer by, for example, extraction as disclosed in JP-A-54-36223. JP6184029 discloses a chemical method to produce p-hydroxybenzyl alcohol by reacting a phenolic compound with a formaldehyde compound in the presence of a cyclic inulooligosaccharide or its alkylation product under basic conditions in a solvent.

In the reaction between phenol and formaldehyde in the presence of a basic catalyst, the production ratio of p-hydroxybenzyl alcohol to o-hydroxybenzyl alcohol is generally no higher than 1.0. Techniques proposed so far for improving the regiospecificity (i.e. increasing the production ratio of p-hydroxybenzyl alcohol) in said reaction system include (1) addition of a polyalkylene ether to the system containing a strongly basic catalyst as disclosed in JP-A-56-16423 and (2) use of an organic nitrogen compound having at least two nitrogen atoms per molecule as a basic catalyst as disclosed in JP-A-56-16424. Nevertheless, the proportion of the para-compound in the resulting mixed hydroxybenzyl alcohol as obtained by these processes is still lower than that of the ortho-compound, i.e., 49% and 47%, respectively. Other attempts to increase the para/ortho production ratio during chemical synthesis of hydroxybenzyl alcohol included the use of cyclodextrin and modified cyclodextrin. EP0386639 discloses a process for preparing p-hydroxybenzyl alcohol or a derivative thereof comprising reacting a phenol and a formaldehyde source under a basic condition, wherein the reaction is carried out in an alcoholic organic solvent in the presence of a crown ether.

Thus, the chemical synthesis of p-HBA faces a number of practical and economical disadvantages. Not only are the chemical reaction schemes complex and expensive to perform, they are also not very regiospecific. As a result, various by-products are formed in addition to the desired para-hydroxybenzyl alcohol. The necessity to remove these by-products further increases the costs of synthesizing para-hydroxybenzyl alcohol of sufficient purity.

It is an object of the present invention to provide a method for the manufacture of p-HBA which overcomes the above disadvantages. In particular, it is an object to provide a method for producing p-HBA from an economically attractive starting material, preferably with a high para position selectivity.

The goal set is met by the unexpected finding that p-HBA can be biologically produced by an organism which has an increased flux in the biosynthesis of aromatic amino acids. It was found that an increased flux in this pathway correlates with a high production of p-HBA. The invention therefore relates to a method for providing p-HBA, comprising the steps of providing a host cell which has an increased flux in the biosynthesis of aromatic amino acids, allowing said host cell to produce p-HBA and isolating said p-HBA.

A host cell having "an increased flux" as used herein refers to an organism wherein the flow of metabolites in a given biosynthetic pathway is increased as compared to the average metabolic flow observed in host cells of the same type or origin. As will be described below, such a host cell is for instance a spontaneous mutant, a mutant generated by random mutagenesis or a genetically engineered host cell, wherein the aromatic amino acid biosynthetic pathway is more active than would be normally expected.

Aromatic amino acids are L-phenylalanine (Phe), L-tyrosine (Tyr) and L-tryptophan (Trp). The biosynthesis of aromatic amino acids bacteria, fungi and higher plants begins with the synthesis of chorismate, which is an important intermediate for many biosynthetic pathways. The process starts with the condensation of phosphoenolpyruvate (PEP) and erythrose-4-phosphate (E-4-P). The pathway for tryptophan synthesis branches after chorismate synthesis. For the biosynthesis of Phe and Tyr, chorismate undergoes a Claisen rearrangement to form prephenate, which is catalyzed by chorismate mutase (CM; EC 5.4.99.5). In *E. coli,* chorismate mutase exists in two bifunctional complexes, one combined with prephenate dehydrogenase (PDH; EC 1.3.1.121; T-protein encoded by *tyrA*) which eliminates CO₂ and a hydride ion to give hydroxyphenylpyruvate, and the other combined with prephenate dehydratase (PDT; EC 4.2.1.51; P-protein encoded by *pheA*) which eliminates CO₂ and a hydroxide ion to give phenylpyruvate. A transaminase reaction completes the synthesis, giving tyrosine and phenylalanine from the respective ketoacids. Trytophan synthesis is complex and involves five separate steps from chorismate. Glutamate donates an amine group in the first step of the pathway and pyruvate is lost from chorismate. In the next three steps a ribose sugar is added, this eventually contributes to the 5 membered ring of tryptophan. In the last step of the pathway serine serves as the donor of the a carbon amino group of tryptophan. It is believed that the flux in the biosynthesis of aromatic amino acids is primarily regulated at the level of the early biosynthetic steps that are shared by two or all three aromatic acids, i.e. at the level of chorismate and prephenate. The present invention discloses the surprising finding that an increased flux in the synthesis of one or more aromatic acids, reflected for example by elevated level of Phe production by a host cell, correlates with increased p-HBA synthesis. In one embodiment, the host cell has an increased level of Trp production relative to a corresponding wild-type host cell. In a preferred embodiment, a host cell has an increased Tyr and/or Phe production level as compared to a wild-type host cell. More preferably, a host cell has an increased Tyr production level as compared to a wild-type host cell.

The present finding that a (micro)organism is capable of producing p-HBA is unexpected. Whereas it is known that certain para-hydroxylated aromatic compounds, e.g. tyrosine and p-hydroxybenzoate, can be microbially produced at an industrial scale, a process for the (micro-) biological production of p-HBA has heretofore not been disclosed. Furthermore, p-HBA is an industrially more useful compound than p-hydroxybenzoate. As said, p-HBA is used on a large scale for the manufacture of various polymers. In addition, p-HBA is a more interesting and versatile precursor in comparison to p-hydroxybenzoate; an alcohol is easily oxidized to an acid whereas reduction of an acid to an alcohol is more difficult. Also, chemically synthesized p-HBA is at least 10-fold more expensive than p-hydroxybenzoate, and about twice as expensive as phenylalanine.

Although the invention is exemplified below by a method using a bacterial host cell, it will be understood that the teaching provided herein can be applied to various types of host cells, including among others fungi and algae. For industrial purposes it is preferred that a host cell can be easily cultured at relatively low cost. Microbial cells are well established as host cell for the industrial production of a wide variety of products, ranging from small molecules to recombinant proteins. In one embodiment, the invention comprises the use of a microbial host cell, such as a Gram-positive or a Gram-negative bacterium. In a specific aspect, a host cell is an *E.coli,* such as *E.coli* strain NST74. This previously described bacterial strain highly overproduces Phe, and to some extent also Tyr (US patent 4,681,852). Bacterial strain NST74 can be obtained from the ATCC (ATCC31884, Manassas, VA, USA).

Also provided herein is the use of a host cell which has an increased flux in the biosynthesis of aromatic amino acids for the biological manufacture of p-HBA. The host cell can use a renewable carbon source, like glucose or other, economically more attractive hydrocarbons, to synthesize p-HBA. In contrast to chemical synthetic routes, the biological synthesis of p-HBA is highly regiospecific. Thus, with a method of the invention p-HBA can be made at a high level of purity for only a fraction of the expense of conventional chemical synthesis.

A method of the invention comprises the use of a host cell which has an increased flux in the biosynthesis of one or more aromatic amino acids. Suitable host cells for use in the present invention can be selected or generated in various ways. In one embodiment, providing the host cell comprises identifying a host cell which shows an increased production level of aromatic amino acids (e.g. Phe and/or Tyr) as compared to the normal or average production level in that type of cell. For example, a large number of subtypes, strains or species of an organism can be screened for the production of an aromatic amino acid from a fermentable carbon source and at least one subtype, strain or species can be selected which displays an increased flux in the biosynthesis of that amino acid. It is not required that the host cell overproduces all three aromatic amino acids; overproduction of either one is sufficiently indicative that the host cell is capable of producing p-HBA. Methods to quantitate the production level of aromatic amino acids are known in the art. See for instance Fiehn, O., Kopka, J., Trethewey, R.N. and L. Willmitzer (2000) "Identification of uncommon plant metabolites based on calculation of elemental compositions using gas chromatography and quadrupole mass spectrometry." Anal. Chem. 72:3573-3580; Park, N.H. and P.L. Rogers (1986) "L-phenylalanine production in continuous culture using a hyperproducing mutant of Escherichiα coli K-12." Chem. Eng. Commun. 45:185-196.

In one embodiment, an aromatic amino acid is detected by metabolic profiling (also referred to as metabolomics) using information from mass spectrometry (MS) and/or nuclear magnetic resonance (NMR) techniques

According to the invention, a suitable host cell can be a naturally occurring cell or a recombinant host cell that is genetically engineered to (further) increase the flux in aromatic amino acid. Methods to increase the flux in the biosynthetic pathway of aromatic amino acids in a host cell have been described in the art. It is possible to obtain a recombinant host cell with increased aromatic amino acid production by overexpression of one or more key genes in the biosynthesis of one or more aromatic amino acids. The invention therefore relates to a method for the production of p-HBA by a host cell, wherein said host cell comprises a genetic modification of at least one gene affecting, either directly or indirectly, the biosynthesis of an aromatic amino acid.
References which are suitably used to provide a host cell for use in the present invention include Ito et al. (1990) "Improvement in microbial production of L-tyrosine by gene dosage effect of aroL gene encoding shikimate kinase." Agric. Biol. Chem. 54:823-824; Berry, A. (1996) "Improving production of aromatic compounds in Escherichia coli by metabolic engineering." Trends Biotechnol. 14:250-256; Patnaik, R. and J.C. Liao (1994) "Engineering of Escherichia coli central metabolism for aromatic metabolite production with near theoretical yield." Appl. Environ. Microbiol. 60:3903-3908; Backman et al. (1990) "Genetic engineering of metabolic pathways applied to the production of phenylalanine." Ann. N.Y. Acad. Sci. 589:16-24; Mascarenhas et al. (1991) "Deletion of pgi alters trytophan biosynthesis in a genetically engineered strain of Escherichia coli." Appl. Environ. Microbiol. 57:2995-2999; and Tatarko, M. and T. Romeo (2001) "Disruption of a global regulatory gene to enhance central carbon flux into phenylalanine biosynthesis in Escherichia coli." Curr. Microbiol. 43:26-32.

In one embodiment, a host cell is genetically engineered to overproduce tyrosine (Ikeda 2003. Amino acid production processes. P. 1-35. in T. Scheper (Ed.), Advances in Biochemical Engineering/Biotechnology, Vol. 79. Springer-Verlag, Berlin Heidelberg). A host cell displaying an elevated tyrosine level is particularly interesting for use in a method of the invention, since tyrosine can be converted to p-HBA as a side-product in the thiamin biosynthetic pathway (see further below).
As described above, the final step in the biosynthesis of tyrosine in *Escherichia coli* and other enteric bacteria is the transamination of p-hydroxyphenylpyruvate, which is produced in two sequential chemical reactions catalyzed by the enzymes CM and PDH. In *E. coli,* both the CM and PDH activities, are located in a single, bifunctional protein known as the T-protein, which is encoded by the *tyrA* gene. In a preferred embodiment of the present invention, a host cell overexpresses the T-protein or a related bifunctional protein from another species which has CM and PDH activities. An analogous bifunctional protein in *E. coli*, known as the P-protein encoded by the *pheA* gene, contains both CM and prephenate dehydratase (PDT) activities, and catalyzes the transformation of chorismate into phenylpyruvate in the biosynthetic pathway to phenylalanine. Disruption of the *pheA* gene may be used to (further) enhance the production of tyrosine, because the less chorismate is converted via prephanate to phenylalanine, the more is available for the production of tyrosine, which can subsequently be converted to p-HBA. Accordingly, in one embodiment, a host cell is genetically engineered to disrupt the *pheA* gene. It is to be noted that such mutant ΔpheA host cell requires phenylalanine supplementation in the growth medium.

In another embodiment, a host cell having an increased flux in the biosynthesis of an aromatic amino acid is selected for increased resistance against a toxic analog of an aromatic amino acid. For example, a mutant organism can be selected for resistance against toxic (ffuoro-)analogs of tyrosine. The mutant can be a spontaneous mutant or it can be generated by (random-)mutagenesis. These insensitive mutants often appear to produce high levels of tyrosine (GB 1071935; US3,709,785).
In addition to an increased flux in aromatic amino acid biosynthesis, it is advantageous if a host cell also shows an increased flux in the biosynthesis of the vitamin thiamin. The thiamin biosynthetic pathway involves the separate synthesis of the thiazole and the pyrimidine moieties, which moieties are then coupled to give thiamin phosphate. The thiazole (5-methyl-4-(2-hydroxyethyl)thiazole phosphate) moiety is formed by the oxidative condensation of tyrosine, cysteine and 1-deoxy-D-xylulose-5-phosphate (Begley et al. (1998) Top. Curr. Chem. 195, 93-142; Begley et al. (1999) Arch. Microbiol. 171, 293-300) and p-HBA is a side-product of this reaction (White RH, Biochim Biophys Acta, 1979 Feb 19, 583(1), 55 - 62). Accordingly, a host cell furthermore has an increased flux the in the biosynthesis of thiazole.

In *E.coli*, thiamin biosynthesis requires at least six enzymes: ThiFSGH, IscS, and ThiI (Leonardi and Roach,"Thiamine biosynthesis in Escherichia coli: in vitro reconstitution of the thiazole synthase activity." J Biol Chem. 2004, 23;279(17): 17054-62). At present relatively little is known regarding the contribution of the individual enzymes to the overall reaction catalyzed, but it is believed that the *thiG* en *thiH* genes encode different subunits which are together catalytically active and required for thiazole synthesis. In one embodiment, p-HBA production by a host cell overproducing an aromatic amino acid is further enhanced by metabolic engineering of the thiazole pathway of said host cell, such that more tyrosine is converted to p-HBA.
In one aspect, it comprises the overexpression of one or more of the genes involved in thiazole synthesis, such as the *thiG* and *thiH* genes of *E.coli* or functional homologs thereof showing at least 70%, preferably at least 80% more preferably at least 90% sequence identity under the provision that the encoded proteins are capable of catalyzing the condensation of tyrosine, cysteine and 1-deoxy-D-xylulose-5-phosphate. Of interest for the present invention are the host cells described in international patent application WO-2004106557 which relates to methods for producing thiamin products using a microorganism containing a mutation that causes it to overproduce and release thiamin products into the medium.

As will be understood, the net production level of p-HBA by a host cell is determined not only by the rate of its synthesis but also by the rate of its degradation or conversion into another molecule. For example, p-HBA can be oxidized to an acid or an aldehyde. Since p-HBA can be toxic for a host cell at high concentrations, it is conceivable that a host cell possesses an intrinsic feed-back mechanism to prevent excessive intracellular accumulation of p-HBA. In a method of the invention, it is therefore advantageous to suppress such a feed-back mechanism in a host cell in order to maximize the production of p-HBA. Preferably, a host cell is genetically modified in at least one gene involved in the degradation or conversion of p-HBA. Of course, such a modification can be present in a host cell which is either selected (e.g. a spontaneous mutant) or genetically engineered in order to increase the flux in the biosynthesis of aromatic amino acids, optionally in combination with enhanced thiamine synthesis.

In one embodiment, a host cell displays a reduced dehydrogenase activity towards p-HBA such that the conversion of p-HBA is suppressed. This can be achieved using a specific inhibitor of dehydrogenase activity or by reducing the expression level of a dehydrogenase. A host cell may for instance have a gene disruption in a gene encoding an NADP-dependent alcohol dehydrogenase (ADH) activity. is disrupted. In a specific aspect, the host cell is an E. *coli* host cell wherein the gene encoding the NADP-dependent alcohol dehydrogenase YqhD. Of course, the use of a host cell other than *E. coli* wherein a gene encoding a functional equivalent of the *E.coli* enzyme YqhD is disrupted is also encompassed.

The nucleotide sequence of various genes encoding an enzyme which is advantageously disrupted or knocked-out in a host cell for use in the present invention (like *YqhD* or *pheA*) are available from public databases. A person skilled in the art will be able to apply his common knowledge, e.g. general principles of recombinant DNA technology, to provide a host cell with one or more disrupted genes. The method disclosed by Datsenko and Wanner ("One-step inactivation of chromosomal genes in Escherichia coli K-12 using PCR products."Proc Natl Acad Sci USA. 2000 Jun 6;97(12):6640-5) is of particular use to generate a (bacterial) host cell with one or more disrupted genes.

As will be understood, a host cell may comprise a combination of two or more of the (genetic) modifications described above to maximize p-HBA production. For example, a host cell may overexpress distinct enzymes, e.g. one enhancing L-tyrosine production and one enhancing thiamine synthesis. In another example, multiple genes are disrupted, e.g. the *pheA* gene to block phenylalanine biosynthesis, thereby increasing the flux in tyrosine production, and *YqhD* gene disruption to prevent further metabolism of p-HBA once it is produced. Furthermore, gene disruption(s) may be combined with overexpression of enzyme(s) in the host cell. In one embodiment, a host cell has disrupted *YqhD* and *pheA* genes and overexpresses the *tyrA* and *thiG*/*H* genes.

In a method of the invention, the host cell is allowed to produce p-HBA. Typically, this involves culturing of the host cell under suitable fermentation conditions for a certain period of time. The conditions depend on the type of host cell and will be known by the person skilled in the art. In one embodiment, the host cell is grown as a cell suspension in a liquid culture medium. In another embodiment, the host cell is grown on a solid culture medium, like agar plates. A host cell that is compatible with liquid culturing conditions, e.g. a bacterial cell, is preferred for the production of p-HBA on an industrial scale.

The fermentation medium comprises, among others, a carbon source which can be converted (fermented) by the host cell into p-hydroxybenzyl alcohol. Suitable carbon sources include glucose, lactose, xylose, galactose, succinate, arabinose and the like. Because of the high costs of glucose, an alternative fermentable carbon source is preferred.

The culture medium may furthermore be supplemented with one or more additives that are of benefit for the host cell and/or the production level of p-HBA. These include vitamins, antibiotics, antioxidants, enzyme inhibitors, precursors of p-HBA. It may be advantageous that the culture medium contains a limiting amount of one or more specific salts, such as phosphate and sulphate (Tribe, D. E. (1983) Novel micro-organism and methods. [US 4,681,852]; Drew, S.W. and A.L. Demain (1977) Effect of primary metabolites on secondary metabolism. Ann. Rev. Microbiol. 31:343-356). As is exemplified in the Experimental Section below, it was observed that the characteristics (e.g. pH, phosphate content) of the culture medium can influence the production ofp-HBA by a host cell. In case of the bacterial strain NST74, it was found that at pH 6.5, a three-fold reduction of the phosphate concentration resulted in a nearly 5-fold increase in the level of p-HBA production. Also, at a given phosphate concentration, a rise in pH from 6.5 to 7 enhanced p-HBA production. Thus, a method of the invention may comprise the optimalisation of the culture conditions (such as culture medium, fermentation time) for a specific host cell in order to achieve an optimal yield ofp-HBA. A skilled person will be able to do so in a screening experiment wherein different parameters are varied one-by-one. An example is shown in Table 1.

The last step of a method provided herein comprises the isolation of p-HBA which is produced by the host cell. Considering the physico-chemical properties of p-HBA, it is conceivable that it passively diffuses over the membrane of the host cell into the cell's surroundings, from where it can be collected or isolated. However, the requirement for and existence of an active transporter for p-HBA cannot be excluded. In a specific aspect, a host cell is allowed to produce and secrete p-HBA in a liquid culture medium, which culture medium comprises an organic solvent for p-hydroxybenzyl alcohol. The organic solvent is meant to "capture" the secreted product, such that toxic product accumulation in the cell is minimized. Therefore, the addition of an organic solvent to the culture medium of a host cell can contribute to increased p-HBA production. Suitable solvents include those with a partition coefficient (logP) larger than 3, preferably larger than 3.3, such as diphenylether, cyclooctane, isooctane, and dibutylphtalate (Kobayashi et al., (1998), Microbiology 144:353-359; Aono et al. (1991) Agric. Biol. Chem. 55:1935-1938; Inoue et al. (1991) J. Ferment. Bioeng. 71:194-196). Isooctane is a preferred solvent. As will be understood, the organic solvent that is added to the culture medium to capture p-HBA should be essentially non-toxic for the host cell under the fermentation conditions used. The maximal tolerable concentration will depend on the host cell and solvent used, and can readily be determined by the skilled person without undue burden.

The p-HBA produced can be obtained from the culture supernatant by methods known in the art. These include the distillation of the organic solvent added to the fermentation medium or, in case no organic solvent is used, extraction of p-HBA from the aqueous medium by an organic solvent, e.g. using ethyl acetate, followed by distillation from the organic phase. It is also possible to use flash chromatography wherein the organic phase comprising the p-HBA is dried over MgSO₄, and concentrated under reduced pressure. The resulting mixture can then be purified by flash chromatography with a 9:1 petroleum ether (bp, 40 to 60°C)-ethyl acetate mixture in order to obtain pure 4-hydroxybenzylalcohol. Flash chromatography is a widely used preparative technique for separating a variety of primarily organic compounds quickly and cost-effectively. Typical flash chromatography media in the market today is irregular silica particle media.

The invention is exemplified by the experimental section below.

### LEGENDS TO THE FIGURES

**Figure 1:** Time course of the production of p-HBA in the medium by a Phe-overproducing (filled circles) and a wild-type (open circles) bacterial host cell. Host cells were cultured in a fermentation medium pH 6.5 comprising glucose as carbon source and 13.2 mM phosphate. Samples were analyzed for p-HBA by GC-MS.
**Figure 2:** Time course of p-HBA (filled symbols) and Phe (open symbols) production by the Phe-overproducing *E.coli* strain NST74. Circles represent the conditions of Exp. 1; triangles Exp. 6 and diamonds represent Exp. 10 (see Table 1).
**Figure 3:** Correlation between Phe production and p-HBA production by a host cell. Shown are results obtained in a wild-type host cell (triangles) and a host cell overproducing Phe (circles). Different data points represent samples from various fermentation experiments.

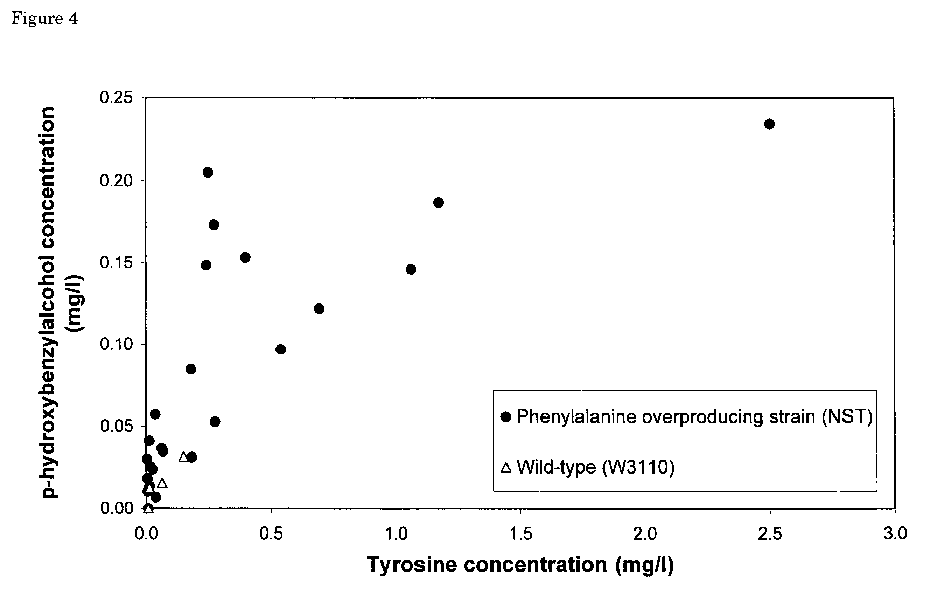

**Figure 4:** Correlation between Tyr production and p-HBA production by a host cell. Shown are results obtained in a wild-type host cell (triangles) and a host cell overproducing Phe (circles). Different data points represent samples from various fermentation experiments.

### EXPERIMENTAL SECTION

### Host cells and culturing conditions

*E. coli* NST 74, the phenylalanine overproducing strain, and E. *coli* W3110, the wild-type strain, were obtained from the ATCC (ATCC31884, Manassas, VA, USA). Cultures were grown batch at 30°C in a Bioflow II (New Brunswick Scientific) bioreactor containing 2 liters of MMT12 medium 14 with 30 g/l glucose as carbon source. The culture was inoculated with 5 % (v/v) of a preculture grown for 16 hours on the same medium (200 rpm; 30°C). A constant pH (pH 6.5) was maintained by the automatic titration with 4 M KOH and 1 M HCl. The oxygen tension was maintained at 30 % by automatic increase of the stirring speed in the fermentor. Samples were taken from the bioreactor after 16, 24, 40, 48 hours, and immediately quenched at -45°C in methanol as previously described by de Koning and van Dam (Anal. Biochem. 1992, 204, 118-23). Variations in this standard fermentation protocol were introduced by changing one of the default conditions, resulting in a screening experiment to find fermentation conditions optimal for p-HBA production (see Table 1). The variations involved the phosphate concentration of the culture medium (13.2 versus 4.4 mM), the pH of the culture medium (pH 6.5 versus pH 7.0), the carbon source (glucose versus succinate), the oxygen concentration (30 versus 2%) and the host cell used (Phe-overproducing strain NST74 *E.coli* versus wild-type).
Experiment 1 is the standard fermentation protocol involving NST74 cultured at 30% oxygen in a fermentation medium pH 6.5, 13.2 mM phosphate using glucose as a carbon source. Deviations from these default conditions, together with the numbering of the experiments, are listed in Table 1.

**Table 1.**

| Sample Nr. | Fermentation times (hour) | Carbon source | Phosphate concentration* | Oxygen (%) | pH | Strain |
|---|---|---|---|---|---|---|
| 1.1 | 16 | Glucose | 1 | 30 | 6.5 | NST 74 |
| 1.2 | 24 | | | | | |
| 1.3 | 40 | | | | | |
| 1.4 | 48 | | | | | |
| 2.2 | 32 | Glucose | 1 | 30 | 6.5 | NST 74 |
| 3.3 | 40 | Glucose | 1 | 2 | 6.5 | NST 74 |
| 4.1 | 16 | Glucose | 1 | unknown | 6.5 | NST 74 |
| 4.2 | 24 | | | | | |
| 4.3 | 40 | | | | | |
| 4.5 | 64 | | | | | |
| 5.1 | 16 | Glucose | 3 | 30 | 6.5 | NST 74 |
| 5.2 | 24 | | | | | |
| 5.3 | 40 | | | | | |
| 5.4 | 48 | | | | | |
| 6.1 | 16 | Glucose | 1/3 | 30 | 6.5 | NST 74 |
| 6.2 | 24 | | | | | |
| 6.3 | 40 | | | | | |
| 7.2 | 24 | Succinate | 1 | 30 | 6.5 | NST 74 |
| 7.3 | 40 | | | | | |
| 7.4 | 48 | | | | | |
| 9.1 | 16 | Glucose | 1 | 30 | 6.5 | W3110 |
| 9.2 | 24 | | | | | |
| 9.3 | 40 | | | | | |
| 9.4 | 48 | | | | | |
| 10.1 | 16 | Glucose | 1 | 30 | 7 | NST 74 |
| 10.2 | 24 | | | | | |
| 10.3 | 40 | | | | | |
| 10.4 | 48 | | | | | |
| | | | | | | |

| | | | | | | |
|---|---|---|---|---|---|---|
| * Phosphate relative to the reference concentration in experiment 1 (13.2mM). | | | | | | |

The intracellular metabolites were extracted from the samples by chloroform extraction, as described by de Koning and van Dam. Appropriate internal standards were added and the samples were lyophilized.

### Detection of metabolites by GC-MS

Lyophilized samples were derivatized using a solution of ethoxyamine hydrochloride in pyridine as the oximation reagent followed by silylation with N-trimethyl-N-trimethylsilylacetamide principally as previously described (Fiehn, O.; Kopka, J.; Trethewey, R. N.; Willmitzer, L. Analytical Chemistry 2000, 72, 3573-80). 1 µl aliquots of the derivatized samples were injected in splitless mode on a HP5-MS 30m x 0.25 mm x 0.25 µm capillary column using a temperature gradient from 70 °C to 325 °C at a rate of 15 °C/min. Gas chromatographic analyses were performed using a 6890 gas chromatograph (Agilent Technologies, Palo Alto, CA, USA). A Pegasus III time-of-flight (TOF) mass spectrometer (LECO Corp., St. Joseph, MI, USA) was used as detector. Detection was performed using MS detection in electron impact (EI) mode (70 eV). This method was used to detect and quantitate the levels of p-HBA, phenylalanine and tyrosine. It allowed to distinguish between p-HBA and o-HBA.

### Results

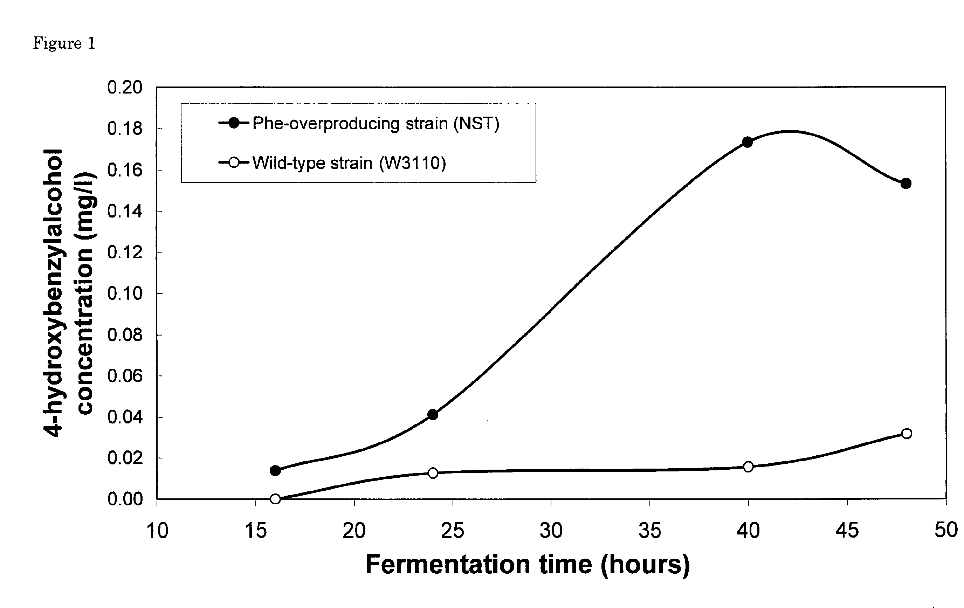

Figure 1 shows the production levels of p-HBA in the medium by a Phe-overproducing host cell and a wild-type host cell. The graphs represent Experiments 1 and 9 of Table 1. Clearly, p-HBA production in the host cell overproducing an aromatic acid is increased as compared to the wild-type host cell. No ortho-HBA could be detected.

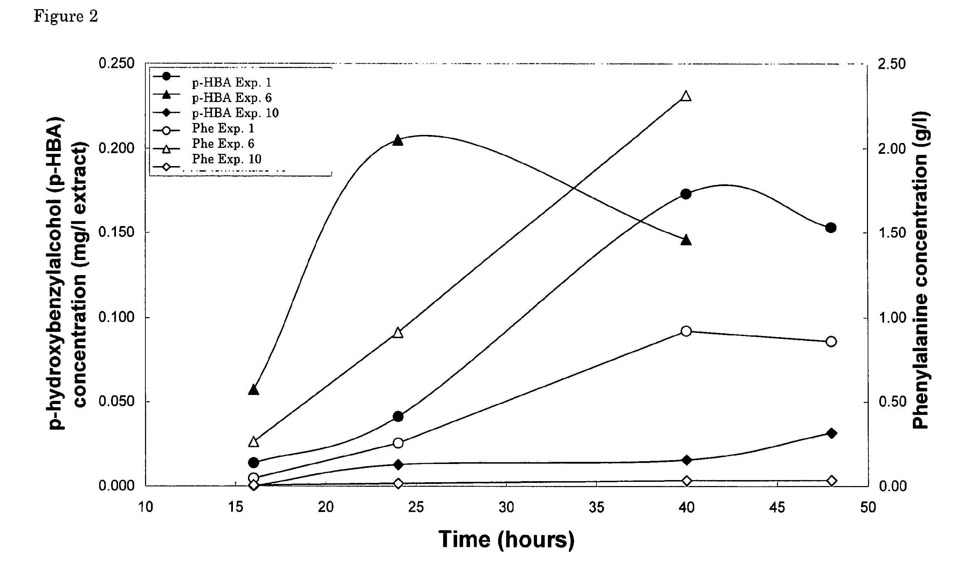

Figure 2 shows the p-HBA and Phe production levels in Experiments 1, 6 and 10 as defined in Table 1. They all involve the Phe-overproducing *E.coli* strain NST74. Experiment 6 uses a 3-fold lower phosphate concentration as compared to the default fermentation procedure of Exp. 1. In Exp. 10 the pH of the fermentation medium is increased from 6.5 to 7.0.

The correlation between Phe and p-HBA production is evident from all three Experiments. Furthermore, lowering the phosphate content in Exp. 6 from 13.2 mM to 4.4 mM results in an increased aromatic acid synthesis and highly increased p-HBA synthesis between approximately 25 and 35 hours of fermentation. A small increase in pH in Exp. 10 is also beneficial for p-HBA production.

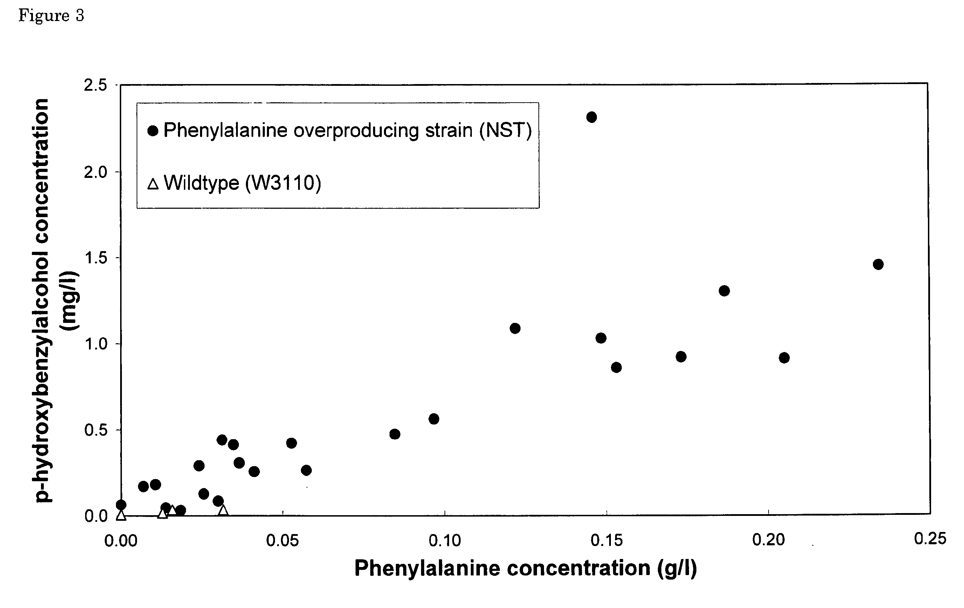

The correlation between aromatic amino acid production and p-HBA production by a host cell is further exemplified in Figures 3 (Phe) and 4 (Tyr).

## Claims

1. A method for providing p-hydroxybenzylalcohol (p-HBA), comprising the steps of:
- providing a host cell which has an increased flux in the biosynthesis of at least one aromatic amino acid;
- allowing said host cell to produce p-HBA; and
- isolating said p-HBA.

2. Method according to claim 1, wherein providing said host cell comprises the identification of an organism which shows an increased production level of L-phenylalanine and/or L-tyrosine.

3. Method according to claim 1 or 2, wherein providing said host cell comprises the genetic modification of at least one gene affecting directly or indirectly the biosynthesis of an aromatic amino acid.

4. Method according to claim 3, wherein said genetic modification comprises the overexpression of a gene encoding a bifunctional protein having chorismate mutase (CM; EC 5.4.99.5) and prephenate dehydrogenase (PDH; EC 1.3.1.12) activities, such as *E.coli* tyrA and/or the disruption of a gene encoding a bifunctional protein having chorismate mutase (CM; EC 5.4.99.5) and prephenate dehydratase (PDT; EC 4.2.1.51) activities, such as *E.coli* pheA.

5. Method according to any one of claims 1 to 4, wherein providing said host cell comprises selecting an organism which has an increased resistance against a toxic analog of an aromatic amino acid.

6. Method according to any one of claims 1 to 5, wherein said host cell furthermore has an increased flux the in the biosynthesis of thiazole.

7. Method according to claim 6, wherein the host cell overexpresses the *thiG* and *thiH* genes of *E.coli* or functional homologs thereof.

8. Method according to any one of claims 1 to 7, wherein said host cell furthermore displays a reduced p-HBA dehydrogenase activity.

9. Method according to claim 8, wherein said host cell has a gene disruption in a gene encoding an NADP-dependent alcohol dehydrogenase activity, preferably wherein the alcohol dehydrogenase YqhD of *E.coli* or a functional equivalent thereof is disrupted.

10. Method according to any one of claims 1 to 9, wherein said host cell is a microbial host cell, preferably a bacterial or a yeast host cell.

11. Method according to claim 10, wherein said host cell is *E. coli,* preferably *E.coli* strain NST74.

12. Method according to any one of claims 1 to 11, wherein said host cell is cultured in a liquid culture medium comprising an organic solvent to capture said p-HBA after it has been produced by the host cell.

13. Use of a host cell which has an increased flux in the biosynthesis of an aromatic amino acid for the biological / microbial manufacture of p-HBA.
